# EUROPEAN PATENT SPECIFICATION

(11) **EP 3 315 084 B1**
(45) Date of publication and mention of the grant of the patent: **13.03.2019**
(21) Application number: 17199402.3
(22) Date of filing: 31.10.2017
(51) Int. Cl.: A61B 17/24, A61B 34/20, A61B 90/00

(54) **RIGID ENT TOOL**
STARRES HNO-WERKZEUG
OUTIL RIGIDE POUR ORL

(30) Priority: 01.11.2016 US 201662415648 P; 02.10.2017 US 201715722329
(43) Date of publication of application: 02.05.2018
(73) Proprietor: Biosense Webster (Israel) Ltd., 2066717 Yokneam (IL)
(72) Inventor: GOVARI, Assaf, 2066717 Yokneam (IL); GLINER, Vadim, 2066717 Yokneam (IL); ALGAWI, Yehuda, 2066717 Yokneam (IL)
(74) Representative: Carpmaels & Ransford LLP

(56) References cited:
- US-A1- 2010 210 939
- US-A1- 2012 172 842
- US-A1- 2012 197 109
- US-A1- 2013 169 272
- US-A1- 2014 266 205

## Description

### FIELD OF THE INVENTION

This invention relates generally to surgical tools, and specifically to a rigid surgical tool which is tracked while in a patient.

### BACKGROUND OF THE INVENTION

In a surgical procedure where a tool, such as an ENT (ear, nose and throat) tool, is inserted into a patient, it is important to track the inserted portion of the tool. The following patents and patent applications provide methods for performing such tracking.

U.S. Patent Application 2006/0004286 to Chang et al. describes systems for performing image guided interventional and surgical procedures, including various procedures to treat sinusitis and other disorders of the paranasal sinuses, ears, nose or throat.

U.S. Patent Application 2012/0143029 to Silverstein et al. describes a guidance system for assisting with the insertion of a needle into a patient body is disclosed. The guidance system may have an imaging device including a probe for producing an image of an internal body portion target, such as a vessel. One or more sensors are included with the probe. The sensors sense a detectable characteristic related to the needle, such as a magnetic field of a magnet included with the needle.

U.S. Patent Application 2012/0226100 to Greenburg et al. describes a system for extending the visual capabilities and working channel of a bronchoscope. The system includes a probe having optic and/or tracking capabilities at a distal tip of the bronchoscope, and that is capable of being advanced through the working channel of a standard bronchoscope.

U.S. Patent Application 2014/0066901 to Dinger, III et al. describes an instrument for treating an anatomical opening such as a paranasal sinus. The application refers to an image guidance navigation system used for positioning a therapeutic component.

U.S. Patent 6,321,109 to Ben-Haim et al. describes a method of excavating tissue in a body. The patent refers to a position sensing device, which senses the instantaneous position of the tip of a catheter. The position sensor is stated to be an AC magnetic field receiver, which senses an AC magnetic field generated by a transmitter.

U.S. Patent Application 2008/0275483 to Makower et al. describes navigation devices for use in conjunction with image guidance or navigation systems and hand held devices.

U.S. Patent 8,721,591 to Chang et al. describes apparatus for dilating ostia of paranasal sinuses. The apparatus may include sensors which are electromagnetic location sensors.

U.S. Patent 5,071,409 to Rosenberg describes a plunger which includes a position sensor for sensing the position of a piston within a cylinder. The position sensor may be in the form of a magnetic core carried by the plunger.

US 2012/0172842 A1 discloses an elongate medical device having an axis comprises an inner liner, a jacket radially outward of the liner, a braid comprising metal embedded in the jacket, a sensor, and at least one wire electrically connected to said sensor. The at least one wire is one of: embedded in the jacket and optionally disposed helically around the braid; extending longitudinally within a tube which extends generally parallel to the device axis and wherein the tube is embedded in the jacket; and disposed within a lumen, wherein the lumen extends longitudinally within the jacket.

US 2014/0266205 A1 discloses an MRl-compatible intrabody device which includes an elongated flexible shaft having a distal end portion, an opposite proximal end portion, an electrical connector interface configured to be in electrical communication with an MRI scanner, and a tracking coil assembly adjacent the shaft distal end portion. The tracking coil assembly includes a first spool having opposing first and second end walls and a second spool having opposing third and fourth end walls. The second spool is in end-to-end relationship with the first spool such that the second and third end walls are in adjacent, spaced-apart relationship. A tracking coil is wound around the first spool, and a coaxial cable is wound around the second spool. An outer conductor of the coaxial cable is connected to one end of the tracking

coil and the inner conductor of the coaxial cable is connected to an opposite end of the tracking coil.

U.S. Patent Application 2010/0210939 describes a suction device with tracking coils positioned near the tip.

### SUMMARY OF THE INVENTION

An embodiment of the present invention provides an ENT apparatus for performing a nasal sinus procedure, including:
a rigid tube enclosing a lumen, configured to be inserted into a cavity of a human patient;
a groove formed in a distal end of the tube;
a conductive coil of insulated wiring wound in the groove; and
a sleeve covering the rigid tube so as to completely enclose the conductive coil.

In a disclosed embodiment the apparatus includes a channel, connected to the groove and formed on the tube, and one or more conductors laid in the channel connected to ends of the conductive coil. Typically the sleeve is configured to cover the channel so as to completely enclose the one or more conductors. The distal end may be chamfered, and the sleeve connects to the chamfered distal end so that an outer surface of the sleeve and the chamfered distal end form a continuous smooth surface.

In a further disclosed embodiment a handle is connected to a proximal end of the tube. Typically, the handle includes circuitry connected to the conductive coil so as to receive signals generated therein.

There is further provided, according to an embodiment of the present invention, a method of providing an ENT apparatus for performing a nasal sinus procedure, including:
providing a rigid tube enclosing a lumen, the rigid tube being configured to be inserted into a cavity of a human patient;
forming a groove in a distal end of the tube;
winding a conductive coil of insulated wiring in the groove; and
covering the rigid tube with a sleeve so as to completely enclose the conductive coil.

The present disclosure will be more fully understood from the following detailed description of the embodiments thereof, taken together with the drawings, in which:

### BRIEF DESCRIPTION OF THE DRAWINGS

Fig. 1 is a schematic illustration of an ENT (ear, nose, and throat) system, according to an embodiment of the present invention; and
Fig. 2 and Fig. 3 are respectively schematic diagrams of an ENT suction tool and of a cross-section of a distal portion of a tube of the tool, according to an embodiment of the present invention.

### DETAILED DESCRIPTION OF EMBODIMENTS

### Overview

It is important to track the distal end of an ENT (ear, nose, and throat) tool used in an ENT procedure, because of the proximity of the end to sensitive areas such as the eyes, optic nerves, and brain. The inventors have found that even though the distal end may be tracked by attaching a tracking sensor to the proximal end of an ENT tool, and adding a vector to the proximal end location, in practice errors are associated with the added vector making the generated distal end value unacceptable.

Embodiments of the present invention form a rigid ENT tool with a magnetic tracking sensor, in this case a single coil, built into the tool. The tool is typically a rigid suction tool, but the concepts described herein can be applied to other rigid tools such as cutters and graspers.

A circular groove is cut into the distal end of the basic tool, and a coil is wound in the groove. A channel is also cut from the circular groove, up to the proximal end of the tool. Conductors connecting the coil to the proximal end are positioned in the channel. The channel and the groove are then covered by a metal sleeve, so that the basic tool external surface returns to its initial un-indented form.

### Detailed Description

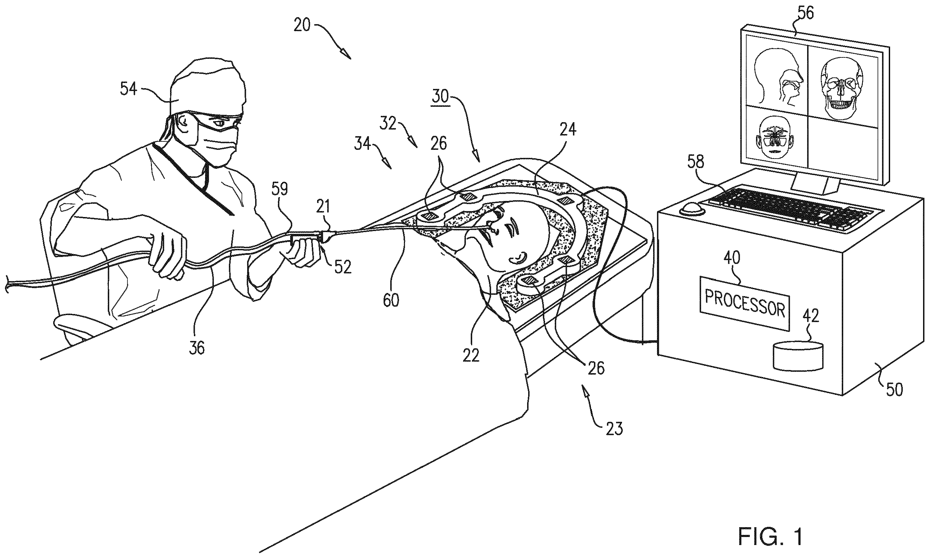

Reference is now made to Fig. 1, which is a schematic illustration of an ENT (ear, nose, and throat) system 20, according to an embodiment of the present invention. In the following description system 20 is assumed to be used to perform a nasal sinus procedure on a patient 22, and during the procedure a suction tool 21 is used. As is described in more detail below, tool 21 comprises a magnetic sensor 32 at its distal tip, and the sensor is tracked during the procedure by a magnetic tracking system 23. For the tracking to be effective, in system 20 frames of reference of a CT (computerized tomography) image of patient 22 and of magnetic tracking system 23, are registered. The method of registration used may comprise any registration method known in the art.

While the CT image may typically comprise a magnetic resonance imaging (MRI) image or a fluoroscopic image, in the description herein the image is assumed to comprise, by way of example, a fluoroscopic CT image,

Prior to and during the sinus procedure, a magnetic radiator assembly 24, comprised in the magnetic tracking system, is positioned in proximity to the patient's head. Assembly 24 comprises magnetic field radiators 26 which are fixed in position and which transmit alternating sinusoidal magnetic fields into a region 30 wherein the head of patient 22 is located. By way of example, radiators 26 of assembly 24 are arranged in an approximately horseshoe shape around the head of patient 22. However, alternate configurations for the radiators of assembly 24 will be apparent to those having ordinary skill in the art, and all such configurations are assumed to be comprised within the scope of the present invention. The Carto® system produced by Biosense Webster, of Diamond Bar, CA, uses a tracking system similar to that described herein for finding the location and orientation of a coil in a region irradiated by magnetic fields.

Suction tool 21 comprises a probe handle 52 which is at the proximal end of the tool, and the suction tool also comprises a rigid tube 60, which is at the distal end of the tool. Handle 52 allows a physician 54 to manipulate the tool. Flexible tubing 59 connects to handle 52, the tubing permitting drainage of fluid through a lumen 34 of tube 60. In addition cabling 36 is connected to handle 52, the cabling enabling power to be transferred to elements in the handle, as well as enabling signals, originating in sensor 32, to be conveyed from the handle.

Elements of system 20, including radiators 26, are controlled by system processor 40. The processor is also configured to receive the signals originating in sensor 32, and process the signals to derive location and orientation values for the sensor. Processor 40 may be mounted in a console 50, which comprises operating controls 58 that typically include a keypad and/or a pointing device such as a mouse or trackball. Console 50 connects to the radiators via a cable and/or wirelessly. Physician 54 uses operating controls 58 to interact with the processor while performing the ENT procedure using system 20. While performing the procedure, the processor may present results of the procedure on a screen 56.

Processor 40 uses software stored in a memory 42 to operate system 20. The software may be downloaded to processor 40 in electronic form, over a network, for example, or it may, alternatively or additionally, be provided and/or stored on non-transitory tangible media, such as magnetic, optical, or electronic memory.

Processor 40 uses the software, *inter alia,* to operate magnetic radiators 26 of assembly 24, and to analyze the signals received from sensor 32. As stated above the radiators transmit sinusoidal alternating magnetic fields of different frequencies into region 30, including the head of patient 22, and the fields from the radiators induce signals in sensor 32.

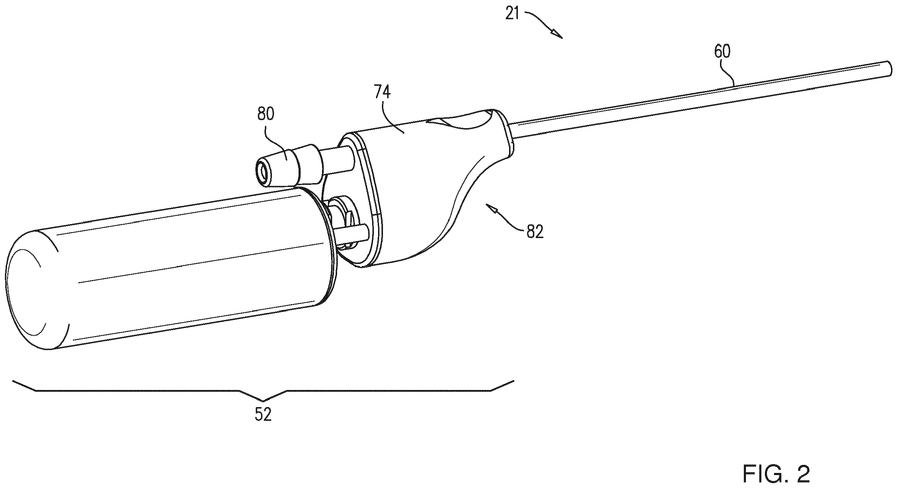

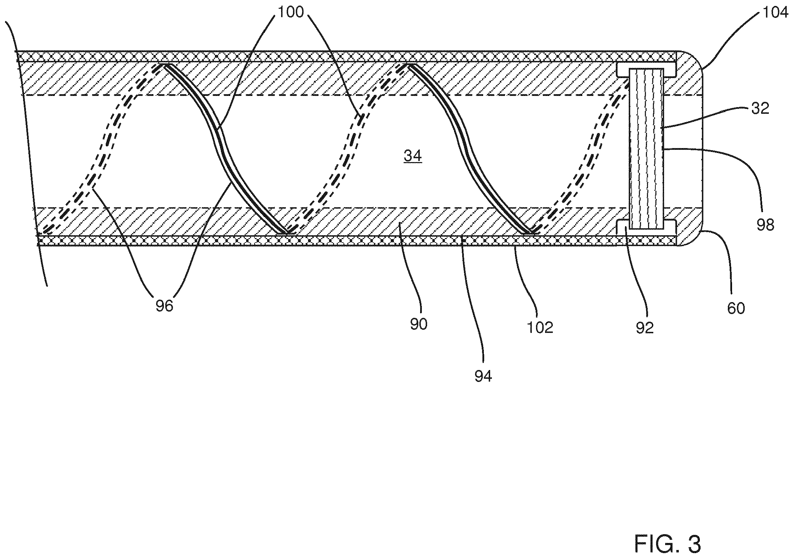

Fig. 2 and Fig. 3 are respectively schematic diagrams of ENT suction tool 21 and of a cross-section of a distal portion of rigid tube 60 of the tool, according to an embodiment of the present invention. As is shown in Fig. 2 rigid, inflexible, tube 60 is formed as a distal end of tool 21. Tube 60 is fixedly attached to a casing 74, which typically encloses circuitry 82 coupled to sensor 32, and which forms part of handle 52. The circuitry typically includes a pre-amplifier which receives signals from sensor 32, and which converts the signals to a form that may be conveyed, via cabling 36, to processor 40 without introducing noise into the signal. A tubular fluid connection 80 is also fixedly attached to casing 74, and the connection connects to tubing 59 (Fig. 1) and is configured so that during the ENT procedure body fluids may drain through lumen 34 of tube 60, and connection 80, without leaking into casing 74.

To operate efficiently, tube 60 has to meet a number of sometimes conflicting constraints. Since tube 60 is to act as a suction device, its interior lumen should be as large as possible, and the lumen should have no internal obstructions which could impede the flow of material in the lumen. However, since the tube is to be used in an internal procedure on a patient, the outside dimensions of the tube should be as small as possible so as to reduce any trauma caused by the entry of the tube into the patient. In addition, also to reduce the chance of trauma, the external surface of the tube should be smooth, without indentations or protuberances. Since the tube is to be used in surgery, surfaces of the tube that may contact a patient should be biocompatible, and the tube itself should be able to be sterilized, for example in an autoclave.

Fig. 3 illustrates how tube 60 is constructed to satisfy the constraints listed above. Tube 60 comprises a cylindrical biocompatible conduit 90, also herein termed tube 90, which encloses lumen 34. A circular groove 92 is formed in an outer surface 94 of the conduit, at the distal tip of the conduit. A channel 96 is formed in the outer surface, leading from groove 92 to casing 74. By way of example, in the embodiment illustrated in Fig. 3, channel 96 is formed as a helical channel, but in other embodiments the channel may be any convenient shape formed in the outer surface, including a straight line, connecting groove 92 to casing 74.

A coil 98 of insulated wiring is wound in groove 92, the coil acting as sensor 32. Ends of the coil are connected to a pair of insulated conductors 100, which are laid in channel 96, and which connect the ends of the coil to circuitry 82. In one embodiment the wiring has a diameter of approximately 40 µm, the groove has a depth of approximately 100 µm, and the channel has a semicircular cross-section with a diameter of approximately 100 µm.

Once coil 98 and conductors 100 have been formed and positioned as described above, channel 96 and groove 92 are covered by a rigid cylindrical biocompatible metal sleeve 102 so that the conductors of the channel and the groove are completely enclosed. The sleeve is typically an interference fit to the external surface of tube 90. In one embodiment the sleeve and tube 90 are constructed from stainless steel or titanium, and after inserting the sleeve over tube 90, the two are welded together. A distal tip 104 of conduit 90 may then be chamfered, as shown in Fig. 3, so that the outer surface of tube 60, comprising the outer surface of sleeve 102 and an external distal part of conduit 90, i.e., chamfered distal tip 104, forms a continuous, smooth, and non-indented surface.

In a disclosed embodiment sleeve 102 has an external diameter of 3.57 mm and an internal diameter of 3.4 mm, and conduit 90 has an external diameter of 3.4 mm and an internal diameter of 3.1 mm.

While the description above has generally referred to a suction tool, it will be understood that the concepts described herein may be applied, *mutatis mutandis,* to other tube based tools, as well as to other tools such as a shaver and a microdebrider.

It will be appreciated that the embodiments described above are cited by way of example, and that the present invention is not limited to what has been particularly shown and described hereinabove. Rather, the scope of the present invention is defined by appended claims.

## Claims

1. An ENT apparatus (21) for performing a nasal sinus procedure, comprising:
a rigid tube (60) enclosing a lumen (34), configured to be inserted into a cavity of a human patient (22);
a groove (92) formed in a distal end of the tube;
a conductive coil (98) of insulated wiring wound in the groove; and
a sleeve (102) covering the rigid tube so as to completely enclose the conductive coil.

2. The apparatus according to claim 1, and comprising a channel (96), connected to the groove and formed on the tube, and one or more conductors (100) laid in the channel connected to ends of the conductive coil.

3. The apparatus according to claim 2, wherein the sleeve is configured to cover the channel so as to completely enclose the one or more conductors.

4. The apparatus according to claim 3, wherein the distal end is chamfered, and wherein the sleeve connects to the chamfered distal end so that an outer surface of the sleeve and the chamfered distal end form a continuous smooth surface.

5. The apparatus according to claim 1, and comprising a handle (52) connected to a proximal end of the tube.

6. The apparatus according to claim 5, wherein the handle comprises circuitry (82) connected to the conductive coil so as to receive signals generated therein.

7. A method of providing an ENT apparatus for performing a nasal sinus procedure, comprising:
providing a rigid tube (60) enclosing a lumen (34), the rigid tube being configured to be inserted into a cavity of a human patient (22);
forming a groove (92) in a distal end of the tube;
winding a conductive coil (92) of insulated wiring in the groove; and
covering the rigid tube with a sleeve (102) so as to completely enclose the conductive coil.

8. The method according to claim 7, and comprising a forming a channel (96), connected to the groove, on the tube, and laying one or more conductors (100), connected to ends of the conductive coil, in the channel.

9. The method according to claim 8, wherein the sleeve is configured to cover the channel so as to completely enclose the one or more conductors.

10. The method according to claim 9, wherein the distal end is chamfered, and wherein the sleeve connects to the chamfered distal end so that an outer surface of the sleeve and the chamfered distal end form a continuous smooth surface.

11. The method according to claim 7, and comprising a handle (52) connected to a proximal end of the tube.

12. The method according to claim 11, wherein the handle comprises circuitry (82) connected to the conductive coil so as to receive signals generated therein.

## Patentansprüche

1. HNO-Vorrichtung (21) zum Durchführen von Nasen-Sinus-Eingriffen, umfassend:
ein starres Rohr (60), das ein Lumen (34) umschließt, das ausgestaltet ist, um in einen Hohlraum eines menschlichen Patienten (22) eingeführt zu werden;
eine Rille (92), die in einem distalen Ende des Rohrs gebildet ist;
eine leitfähige Spule (98) mit isoliertem Draht, der in die Rille gewickelt ist; und
eine Manschette (102), die das starre Rohr bedeckt, um so die leitfähige Spule vollständig zu umschließen.

2. Vorrichtung nach Anspruch 1, und umfassend einen Kanal (96), der mit der Rille verbunden und auf dem Rohr gebildet ist, und einen oder mehrere Leiter (100), der/die in dem Kanal liegt bzw. liegen und mit Enden der leitfähigen Spule verbunden ist bzw. sind.

3. Vorrichtung nach Anspruch 2, wobei die Manschette ausgestaltet ist, um den Kanal zu bedecken, um so den einen oder die mehreren Leiter vollständig zu umschließen.

4. Vorrichtung nach Anspruch 3, wobei das distale Ende abgeschrägt ist, und wobei die Manschette mit dem abgeschrägten distalen Ende derart verbunden ist, dass eine Außenfläche der Manschette und das abgeschrägte distale Ende eine kontinuierliche glatte Oberfläche bilden.

5. Vorrichtung nach Anspruch 1, und umfassend einen Griff (52), der mit einem proximalen Ende des Rohrs verbunden ist.

6. Vorrichtung nach Anspruch 5, wobei der Griff Schaltkreis(e) (82) umfasst, der/die mit der leitfähigen Spule verbunden ist bzw. sind, um so darin generierte Signale zu empfangen.

7. Verfahren zum Bereitstellen einer HNO-Vorrichtung zum Durchführen von Nasen-Sinus-Eingriffen, umfassend:
Bereitstellen eines starren Rohrs (60), das ein Lumen (34) umschließt, wobei das starre Rohr ausgestaltet ist, um in einen Hohlraum eines menschlichen Patienten (22) eingeführt zu werden;
Bilden einer Rille (92) in einem distalen Ende des Rohrs;
Wickeln einer leitfähigen Spule (92) des isolierten Drahts in die Rille; und
Bedecken des starren Rohrs mit einer Manschette (102), um so die leitfähige Spule vollständig zu umschließen.

8. Verfahren nach Anspruch 7, und umfassend Bilden eines Kanals (96), der mit der Rille verbunden ist, auf dem Kanal, und Legen von einem oder mehreren Leitern (100), die mit Enden der leitfähigen Spule verbunden sind, in den Kanal.

9. Verfahren nach Anspruch 8, wobei die Manschette ausgestaltet ist, um den Kanal zu bedecken, um so den einen oder die mehreren Leiter vollständig zu umschließen.

10. Verfahren nach Anspruch 9, wobei das distale Ende abgeschrägt ist, und wobei die Manschette mit dem abgeschrägten distalen Ende verbunden ist, so dass eine Außenfläche der Manschette und das abgeschrägte distale Ende eine kontinuierliche glatte Oberfläche bilden.

11. Verfahren nach Anspruch 7, und umfassend einen Griff (52), der mit einem proximalen Ende des Rohrs verbunden ist.

12. Verfahren nach Anspruch 11, wobei der Griff Schaltkreis(e) (82) umfasst, der/die mit der leitfähigen Spule verbunden ist bzw. sind, um so darin generierte Signale zu empfangen.

## Revendications

1. Appareil ORL (21) servant à effectuer une procédure sur un sinus nasal, comprenant :
un tube rigide (60) encerclant une lumière (34), configuré pour être inséré dans une cavité d'un patient humain (22) ;
une rainure (92) formée dans une extrémité distale du tube ;
une bobine conductrice (98) de câblage isolé enroulée dans la rainure ; et
un manchon (102) recouvrant le tube rigide de manière à encercler entièrement la bobine conductrice.

2. Appareil selon la revendication 1, et comprenant un canal (96), raccordé à la rainure et formé sur le tube, et un ou plusieurs conducteurs (100) disposés dans le canal reliés à des extrémités de la bobine conductrice.

3. Appareil selon la revendication 2, dans lequel le manchon est configuré pour recouvrir le canal de manière à encercler entièrement le ou les conducteurs.

4. Appareil selon la revendication 3, dans lequel l'extrémité distale est biseautée, et dans lequel le manchon se raccorde à l'extrémité distale biseautée de telle sorte qu'une surface externe du manchon et l'extrémité distale biseautée forment une surface lisse continue.

5. Appareil selon la revendication 1, et comprenant une poignée (52) raccordée à une extrémité proximale du tube.

6. Appareil selon la revendication 5, dans lequel la poignée comprend un circuit (82) relié à la bobine conductrice de manière à recevoir des signaux générés à l'intérieur de celle-ci.

7. Procédé de fourniture d'un appareil ORL servant à effectuer une procédure sur un sinus nasal, comprenant :
la fourniture d'un tube rigide (60) encerclant une lumière (34), le tube rigide étant configuré pour être inséré dans une cavité d'un patient humain (22) ;
la formation d'une rainure (92) dans une extrémité distale du tube ;
l'enroulement d'une bobine conductrice (92) de câblage isolé dans la rainure ; et
le recouvrement du tube rigide avec un manchon (102) de manière à encercler entièrement la bobine conductrice.

8. Procédé selon la revendication 7, et comprenant une formation d'un canal (96), raccordé à la rainure, sur le tube, et la mise en place d'un ou plusieurs conducteurs (100), reliés à des extrémités de la bobine conductrice, dans le canal.

9. Procédé selon la revendication 8, dans lequel le manchon est configuré pour recouvrir le canal de manière à encercler entièrement le ou les conducteurs.

10. Procédé selon la revendication 9, dans lequel l'extrémité distale est biseautée, et dans lequel le manchon se raccorde à l'extrémité distale biseautée de telle sorte qu'une surface externe du manchon et l'extrémité distale biseautée forment une surface lisse continue.

11. Procédé selon la revendication 7, et comprenant une poignée (52) raccordée à une extrémité proximale du tube.

12. Procédé selon la revendication 11, dans lequel la poignée comprend un circuit (82) relié à la bobine conductrice de manière à recevoir des signaux générés à l'intérieur de celle-ci.
